(19) Europäisches Patentamt
European Patent Office
Office européen des brevets

(11) Publication number: **0 272 238 B1**

(12) **EUROPEAN PATENT SPECIFICATION**

(45) Date of publication of patent specification: **27.01.93**

(51) Int. Cl.⁵: **C07C 209/60**, C07C 211/54

(21) Application number: **87870179.6**

(22) Date of filing: **16.12.87**

(54) **Preparation of substituted aromatic amines.**

(30) Priority: **17.12.86 US 942743**

(43) Date of publication of application:
**22.06.88 Bulletin 88/25**

(45) Publication of the grant of the patent:
**27.01.93 Bulletin 93/04**

(84) Designated Contracting States:
**AT BE CH DE ES FR GB GR IT LI LU NL SE**

(56) References cited:
**GB-A- 1 440 767**

**CHEMICAL ABSTRACTS, vol. 92, no. 18, 5th May 1980, page 95, abstract no. 148522s, Columbus, Ohio, US; B.A. TAGIEV et al.: "Aniline black"**

(73) Proprietor: **Monsanto Company**
**Patent Department 800 North Lindbergh Boulevard**
**St. Louis Missouri 63167-7020(US)**

(72) Inventor: **Genetti, Ralph Albert**
**211 Scenic View Drive**
**Copley Ohio 44321(US)**
Inventor: **Solodar, Arthur John**
**8135 Cornell Court**
**University City Missouri 63130(US)**

(74) Representative: **McLean, Peter et al**
**MONSANTO SERVICES INTERNATIONAL S.A.**
**Avenue de Tervuren 270/272 Letter Box 21**
**B-1150 Brussels(BE)**

**EP 0 272 238 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of substituted aromatic amines. More specifically, the invention relates to the preparation of aminodiphenylamines.

British Patent 1,440,767 describes the direct synthesis of 4-aminodiphenylamine (4-ADPA) by the head-to-tail coupling of aniline by oxidation with ferricyanide followed by hydrogenation. This process has numerous disadvantages. Ferricyanide is an expensive catalyst which is relatively difficult to handle. Additionally, the overall selectivities to 4-ADPA are relatively low, in the range of about 9 to 37% based on aniline consumed after the hydrogenation step.

Other references teach the use of hypochlorite as an amine oxidant to prepare compounds other than the present substituted aromatic amines. USSR Patent 712,425 discloses the polycondensation of aniline in the presence of sodium hypochlorite to prepare aniline black. Aust. J. Chem. 1984, 37, 2013-26 discloses a method of synthesizing benzofurans by the oxidation of 2-nitroanilines with alkaline hypochlorite. Neither of the above references, however teach the use of aromatic amines and a hypochlorite oxidizing agent to prepare the substituted aromatic amines described in detail below.

### Summary of the Invention

A process for the preparation of substituted aromatic amines of the formula

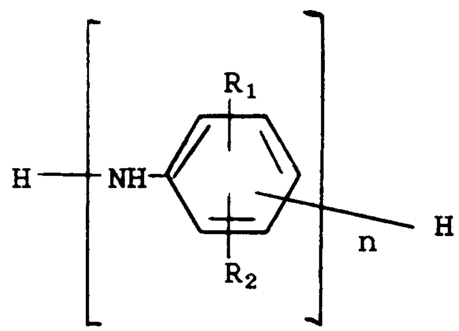

wherein n equals from 2 to 5,

$R_1$ and $R_2$ are either the same or different aliphatic radicals or hydrogen, which comprises (1) contacting one or more primary aromatic amines with a hypohalite oxidizing agent in a mixture containing water and an organic solvent and a base, and (2) reducing the mixture with a reducing agent to form the substituted aromatic amine.

The present process is an improvement over BP 1,440,767, in that the process uses a hypohalite oxidizing agent, rather than ferricyanide, which is less expensive and easier to handle and which provides much higher selectivities to substituted aromatic amines than produced by the methods taught in BP 1,440,767, e.g. in the range of 16 to 91% based on aniline consumed after the hydrogenation step.

### Detailed Description of the Invention

The process involves two steps: the oxidation of a primary aromatic amine and its reduction to form substituted aromatic amines. The substituted amines are oligomers of the primary aromatic amines where n equals from 2 to 5 and $R_1$ and/or $R_2$ are hydrogen or the same or different aliphatic radicals.

In the first step, a primary aromatic amine is oxidized to form a mixture of oxidation products which includes some products where benzene rings are bound by azo linkages. The oxidation step is carried out by mixing the amine or a solution of the amine in a mixture containing water, an organic solvent, an oxidizing agent and a base.

Suitable primary aromatic amines are of the structure

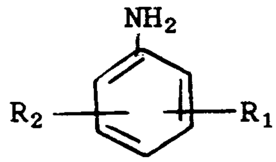

where $R_1$ and $R_2$ are either the same or different aliphatic radicals or hydrogen. Examples of such primary aromatic amines include 2-methyl aniline, 2-ethyl aniline and 2,6-dimethyl aniline or mixtures of such amines. The preferred amine is aniline, a readily available commodity chemical.

The oxidation step is carried out with an oxidizing agent and a base in a mixture containing water and an organic solvent. The organic solvent can be any of a number of organic solvents such as alcohols, e.g. methanol, ethanol, propanol, isopropanol, butanol, 2-butanol; nitriles such as acetonitrile; ethers such as polyethylene glycols; ketones such as methylethyl ketone and acetone; alkanes such as hexane, cyclohexane, heptane, octane and isooctane and other solvents such as tetrahydrofuran and dimethyl formamide.

The ratio of solvent to water in the oxidation step can be varied widely, in the range of 5:95 to 95:5 volume ratio, with the preferred volume ratios being in the range of 40:60 to 60:40. The amount of primary aromatic amine reactant present in the solvent-water solution is very dilute to discourage side reactions, such as polymerization of the substituted amine where n is greater than 5. Typically, the weight ratio of amine to solvent mixture is in the range of 0.001 to 1.20, and the preferred range is 0.01 to 0.1, to provide a dilute enough solution to discourage excessive polymerization but high enough concentration to produce an appreciable amount of substituted amine.

The oxidizing agent is a hypohalite and is typically added as the hypohalite salt such as sodium hypochlorite, sodium hypobromite, potassium hypochlorite, calcium hypochlorite, and magnesium hypochlorite. The preferred hypochlorite salt is sodium hypochlorite since it is readily available as commercial bleach. The amount of oxidizing agent used can vary widely. The smaller the amount used, the greater the selectivity to the substituted aromatic amines and the lower the conversion. The greater the amount used, the higher the conversion of the primary aromatic amine but the lower the selectivity to substituted aromatic amine. Typically, the mole ratio of oxidizing agent to primary amine is in the range of 4:1 to 1:4, with the preferred ratio being about 1:1 to achieve the desired balance of selectivity and conversion.

The reaction mass can be a single homogeneous system or a two-phase system depending on the amount of salt present in the system. If a higher concentration of salt formed from the oxidant and base is present, a two-phase system may occur, requiring more vigorous mixing. However, separation and recovery of the product may be easier in the two-phase system.

The base which is used in the oxidation step can be any of a number of bases, such as sodium hydroxide, potassium hydroxide, ammonium hydroxide, tetramethyl ammonium hydroxide, ammonium sodium carbonate and lithium hydroxide, or a mixture of such bases. The base may be added with the oxidizing agent as a hypochlorite salt, e.g. sodium hydroxide mixed with hypochlorite to form sodium hypochlorite. Sufficient base is added to raise the pH in the range of 10 to 14. The preferred pH is in the range of 11 to 13.

The oxidation step reaction occurs quickly and the reaction time, therefore, can be very short. If the reaction is allowed to continue for a long period of time, a slow reaction of the substituted amine continues, resulting in polymers where n is greater than 5 and a subsequent loss of selectivity. The reaction time is typically in the range of 1 second to 60 minutes, with the preferred range being 12 seconds to 5 minutes.

The oxidation step reaction can be run at relatively low temperatures, with the best results occurring below 35°C. At higher temperatures the substituted amines can react further to form polymers with n greater than 5 and tars, resulting in a loss of selectivity. The preferred reaction temperature range is 20°C to 30°C. The most preferred reaction temperature, because of ease of operation, is room temperature, around 23°C although the reaction temperature may temporarily be hotter than 23°C, due to the exothermic oxidation reaction.

There are many methods of performing the oxidation step. One method is to mix the reactants in a single vessel. Another method is to pump separate streams of the reagents through two legs of a Y-shaped tube such that the streams meet in the center and exit out the third leg of the tube. The reaction time in the Y-shaped reactor is controlled by the rate of pumping in the reactants and the length of the exit tube. Another method is the use of a liquid-liquid extractive reactor, which is used when the salt concentration in the hypohalite phase is such that the final reaction mixture forms two immiscible phases. In this extractive reactor, the heavier, aqueous phase flows into the reactor from the top and the lighter organic phase flows into the reactor from the bottom, effecting good mixing.

The second step of the process to make substituted aromatic amines is to reduce the oxidation product. This results in two reactions. One produces the substituted amines from an unidentified intermediate. The second reduces the azobenzene compounds to the primary aromatic amine, which can be recovered and recycled. The reduction can be carried out by any of many known reductive processes, such as using a hydride such as sodium borohydride or sodium borohydride in conjunction with palladium- or platinum-on-carbon calatyst. The preferred process is catalytic reduction wherein hydrogenation can be effected under pressure in the presence of platinum- or palladium-on-carbon as catalyst. This process is described in detail in "Catalytic Hydrogenation in Organic Synthesis", P. N. Rylander, Academic Press, N.Y. 1979, p 299, which is hereby incorporated by reference.

## Examples

The following examples are for illustration purposes only and in no way limit the scope of this invention.

In the following examples, oxidation is effected as follows:

At room temperature, the base, as a solid, the oxidizing agent and about one-half of the water is added to a round bottomed flask with stirring. After the solids dissolve, about one-half of the solvent, a solution of the aniline and the other one-half of the solvent, and the remaining water are added. The mixture is stirred for the designated reaction time.

Hydrogenation is effected as follows:

The reaction mixture is placed in an autoclave reactor with 80 ml ethanol and 0.2 g of 5% palladium-on-charcoal catalyst. Hydrogen is added to the reactor and the solution is hydrogenated at 50°C and 100 psig (689 kPa) for about 30 minutes.

The resulting mixture is analyzed for aniline by gas chromatographic separation on a 30 meter fused silica column of DB wax-30W using either 1-octanol or dodecane as an internal standard. The separation is temperature programmed from 70° to 220°C at 10°/minute.

The reduction product is analyzed by high pressure liquid chromatography (HPLC) to determine azobenzene, 4-ADPA and 2-ADPA. The analysis is performed on a $C_{18}$ reverse phase (ODS) column using acetonitrile-water as the solvent programmed from 35:65 to 75:25 (volume:volume) acetonitrile:water over 15 minutes at 1.0 ml/minute. Fluoranthene or ρ-nitrochlorobenzene is used as the internal standard. A Hewlett-Packard Diode Array detector set at 282 ± 15 nanometer was employed for peak detection and integration.

## Table 1

Examples 1 through 13, given in Table 1, show the effect of varying the type of organic solvent. In Example 1, 2-propanol gave the highest yield, while ethanol (Example 4) gave the highest selectivity.

## Table 2

Examples 14 through 21, given in Table 2, show the effect of varying the commercial source of the hypohalite (e.g. Purex® bleach, Clorox® bleach, Aldrich, Fisher Scientific and Perma Tech) or the type of hypohalite e.g. sodium hypochlorite (Examples 14 through 20) or calcium hypochlorite (Example 21). In Examples 19 through 21, the base is present with the hypohalite as the salt of the hypohalite. Higher selectivities are achieved (Examples 14 through 18) when additional base is present.

Table 1

Varying Solvent

| Example | Solvent | Selectivity* (%) | Conversion* (%) | Yield* (%) |
|---|---|---|---|---|
| 1 | 2-propanol | 63 | 47 | 29.6 |
| 2 | Acetonitrile | 37 | 32 | 11.8 |
| 3 | Tetrahydrofuran | 43 | 25 | 10.8 |
| 4 | Ethanol | 70 | 37 | 25.9 |
| 5 | 1-Butanol | 18 | 38 | 6.8 |
| 6 | 2-Butanol | 16 | 18 | 2.9 |
| 7 | t-Butanol | 43 | 45 | 19.4 |
| 8 | i-Butanol | 25 | 22 | 5.5 |
| 9 | Propylene glycol | 63 | 29 | 15.4 |
| 10 | 1,3-Propanediol | 49 | 38 | 18.6 |
| 11 | 1,4-butanediol | 45 | 39 | 17.6 |
| 12 | 1,3-butanediol | 54 | 38 | 20.5 |
| 13 | 1-Propanol | 39 | 42 | 16.4 |

Table 1 (continued)

A single phase system where the hypohalite is 6.0 mmoles of 5.25 weight % sodium hypochlorite in water (Clorox® bleach), with the exception of example 6 which used 11.5 mmoles, 20 ml water, 6.0 mmoles of aniline, 7.2 mmoles of NaOH base, 20 ml of the indicated solvent. The reaction time is 5 minutes at room temperature. All of the examples, except example 7 produced only 4-ADPA isomer and no 2-ADPA. The ratio of 4-ADPA to 2-ADPA is 95/5 for example 7.

*Selectivity and conversion are calculated based on aniline ranges recovered after hydrogenation. Yield is selectivity multiplied by conversion.

Table 2

Varying Base

| Example | Aniline (mmoles) | Base (mmoles) | Hypohalite (mmoles) | Selectivity (%) | Conversion (%) | Yield (%) |
|---|---|---|---|---|---|---|
| 14 | 6.0 | 7.2 | 6.0 A | 89 | 35 | 31 |
| 15 | 6.0 | 7.2 | 6.0 B | 79 | 42 | 33 |
| 16 | 6.0 | 7.2 | 6.0 C | 63 | 43 | 27 |
| 17 | 6.0 | 7.2 | 6.0 D | 90 | 36 | 32 |
| 18 | 24.0 | 28.8 | 24.0 E | 70 | 34 | 24 |
| 19 | 12.0 | 0 | 12.0 B | 39 | 39 | 15 |
| 20 | 12.0 | 0 | 12.0 A | 30 | 11 | 3 |
| 21 | 12.5 | 0 | 12.0 F | 33 | 45 | 15 |

**The reaction mass included 20 ml of water, 20 ml of 1-propanol solvent, and NaOH as base. The reaction time is 5 minutes at room temperature. All examples except, example 21 produced only 4-ADPA isomer and no 2-ADPA. The ratio of 4-ADPA to 2-ADPA is 78/22 for example 21. Examples 14-17 were single phase and Examples 18-21 were two-phase reactions.**

Table 2 (continued)

| | |
|---|---|
| A | 5.25 weight % of NaOCl in water (Purex® bleach) |
| B | 5.25 weight % of NaOCl in water (Clorox® bleach) |
| C | 5.25 weight % of NaOCl in water (from Aldrich) |
| D | 5.25 weight % of NaOCl in water (from Fisher Scientific) |
| E | 10.5 weight % of NaOCl in water (from Perma Tech) |
| F | Solid calcium hypochlorite from Fisher Scientific. |

Table 3

Varying Reaction Temperature

| Example | Temperature Range* (°C) | Selectivity (%) | Conversion (%) | Yield (%) |
|---|---|---|---|---|
| 22 | 5-27 | 61 | 54 | 33 |
| 23 | 8-37 | 71 | 52 | 36 |
| 24 | 22-45 | 77 | 50 | 38 |
| 25 | 35-47 | 24 | 74 | 18 |

The reaction run time was 5 min. 85 ml of water, 100 ml of 1-propanol and 60.4 mmole of aniline were charged to the reactor. 58.5 mmoles of 5.25 wt. % of HClO in water (Clorox® Bleach) and 72.0 mmoles of NaOH were then added. All of the examples were two phase and produced only the 4-ADPA isomer.

*The temperature range is determined by a thermometer immersed in the liquid in the reaction flask.

Table 4

Changing other Variables

| Example | Addition Time(min) | Reaction Time(min) | $H_2O$ (ml) | Solvent (ml) | Aniline (mmoles) | Base (mmole) | Hypohalite (mmoles) | Select. (%) | Conv. (%) | Yield (%) |
|---|---|---|---|---|---|---|---|---|---|---|
| 26 | 0 | 5 | 17 | 20 | 12 | 14.4 | 12 | 80 | 38 | 30 |
| 27 | 1 | 5 | 17 | 20 | 12 | 14.4 | 12 | 78 | 34 | 27 |
| 28 | 10 | 5 | 17 | 20 | 12 | 14.4 | 12 | 91 | 35 | 32 |
| 29 | 10 | 5 | 17 | 20 | 24 | 15.2 | 12 | 84 | 18 | 15 |
| 30 | 18 | 5 | 34 | 40 | 24 | 28.8 | 24.4 | 74 | 42 | 31 |
| 31 | 0 | 5 | 20 | 20 | 6 | 7.2 | 6 | 79 | 42 | 33 |
| 32 | 10 | 5 | 20 | 20 | 6 | 7.2 | 6 | 68 | 41 | 28 |
| 33 | 20 | 10 | 24 | 16 | 6 | 6.0 | 12 | 62 | 62 | 38 |
| 34 | 1 | 10 | 24 | 16 | 24 | 24.0 | 12 | 38 | 19 | 7 |
| 35 | 20 | 10 | 24 | 16 | 6 | 6.0 | 6 | 70 | 45 | 32 |
| 36 | 1 | 10 | 26 | 102 | 6 | 6.0 | 18 | 60 | 67 | 40 |
| 37 | 1 | 1 | 26 | 17 | 6 | 24.0 | 18 | 24 | 100 | 24 |
| 38 | 1 | 10 | 24 | 16 | 24 | 24.0 | 6 | 45 | 14 | 6 |
| 39 | 1 | 1 | 8.5 | 34 | 24 | 6.0 | 6 | 37 | 16 | 6 |
| 40 | 20 | 1 | 8.5 | 34 | 6 | 24.0 | 6 | 60 | 47 | 28 |
| 41 | 20 | 1 | 26 | 17 | 24 | 6.0 | 18 | 53 | 33 | 17 |
| 42 | 20 | 10 | 26 | 102 | 24 | 24.0 | 18 | 65 | 36 | 23 |
| 43 | 26 | 13 | 8.5 | 38.5 | 24 | 6.0 | 6 | 66 | 12 | 8 |
| 44 | 26 | 13 | 26 | 17 | 6 | 6.0 | 18 | 56 | 62 | 35 |
| 45 | 10 | 5 | 17 | 20 | 12 | 14.4 | 12 | 54 | 44 | 24 |
| 46 | 10 | 5 | 17 | 20 | 12 | 14.4 | 12.3 | 53 | 39 | 21 |
| 47 | 10 | 5 | 17 | 20 | 12 | 14.4 | 12.3 | 75 | 39 | 29 |

The water, 1-propanol solvent and aniline were charged to the vessel. The base (NaOH) and oxidant (5.25 wt. % HClO in water, sold as Clorox® Bleach) were pumped in during the indicated time period. All of the examples were two phase and produced only the 4-ADPA isomer and no 2-ADPA.

Table 3

Examples 22 through 25 given in Table 3, show the effect of varying reaction temperature. As seen when comparing Example 25 to Examples 22 through 24, temperatures above 35 ° C tend to result in higher

conversion but lower selectivity.

Table 4

Examples 26 through 47 given in Table 4, show the effect of varying addition time, reaction time, solvent to water ratio, aniline, base and hypohalite concentrations.

Example 48 involves the use of sodium hypobromite as the oxidation catalyst. The sodium hypobromite was formed by mixing 0.79 g NaOH, 20 ml water and 0.31 ml liquid bromine, then added to 20 ml of 1-propanol and 0.55 ml aniline and mixed for 8 minutes. The oxidation mixture was reduced as described above. The conversion to 4-ADPA was 50%, the selectivity to 4-ADPA was 56% and the yield was 28%.

**Claims**

1. A process for the preparation of substituted aromatic amines of the formula

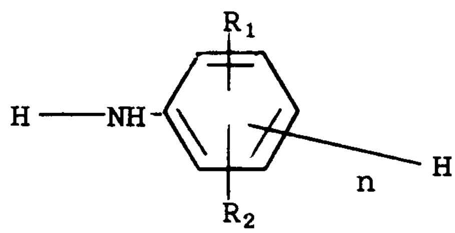

wherein n equals from 2 to 5,

$R_1$ and $R_2$ are either the same or different aliphatic radicals or hydrogen, which comprises (1) contacting one or more primary aromatic amine with a hypohalite oxidizing agent in a mixture containing water, an organic solvent and a base, and (2) reducing the solution with a reducing agent to produce the substituted aromatic amine.

2. The process of Claim 1 wherein the primary aromatic amines are of the structure

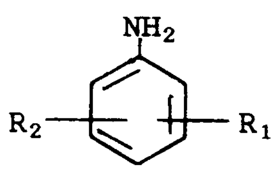

where $R_1$ and $R_2$ are either the same or different aliphatic radicals or hydrogen.

3. The process of Claim 2 wherein the primary aromatic amine is aniline.

4. The process of Claim 1 wherein the oxidizing agent is a hypohalite salt.

5. The process of Claim 3 wherein the organic solvent is selected from the group consisting of methanol, ethanol, 1-propanol, 1-butanol, 2-propanol, 2-butanol, acetonitrile and tetrahydrofuran.

6. The process of Claim 3 wherein the volume ratio of solvent to water is in the range of 5:95 to 95:5.

7. The process of Claim 3 wherein the weight ratio of aniline to the mixture containing water and solvent is in the range of 0.001 to 1.20.

8. The process of Claim 3 wherein the mole ratio of oxidizing agent to primary amine is in the range of 4:1 to 1:4.

9. The process of Claim 3 wherein the mole ratio of base to oxidizing agent is in the range of 1:1 to 4:1.

10. The process of Claim 3 wherein the oxidation step is run at a temperature of less than 35 ° C.

11. The process of Claim 3 wherein the reducing agent is selected from the group consisting of a palladium-on-carbon catalyst and a platinum on-carbon catalyst.

12. The process of Claim 1 wherein the primary aromatic amine is aniline, the organic solvent is an alcohol selected from methanol, ethanol, 1-propanol and 2-propanol and wherein catalytic reduction is used to produce the 4-aminodiphenylamine or 2-aminodiphenylamine or the mixture thereof.

**Patentansprüche**

1. Verfahren zur Herstellung von substituierten aromatischen Aminen der Formel

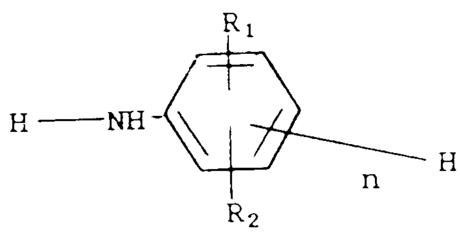

,

worin n 2 bis 5 ist und $R_1$ und $R_2$ entweder gleiche oder verschiedene aliphatische Reste oder Wasserstoff bedeuten, das (1) das Inberührungbringen eines oder mehrerer primärer aromatischer Amine mit einem Hypohalogenit-Oxidationsmittel in einer Wasser, ein organisches Lösungsmittel und eine Base enthaltenden Mischung und (2) das Reduzieren der Lösung mit einem Reduktionsmittel unter Bildung des substituierten aromatischen Amins umfaßt.

2. Verfahren nach Anspruch 1, in dem das primäre aromatische Amin die Formel

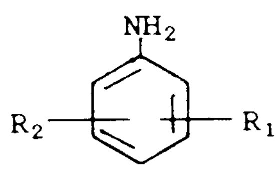

,

worin $R_1$ und $R_2$ entweder die gleichen oder verschiedene aliphatische Reste oder Wasserstoff bedeuten, aufweist.

3. Verfahren nach Anspruch 2, in dem das primäre aromatische Amin Anilin ist.

4. Verfahren nach Anspruch 1, in dem das Oxidationsmittel ein Hypohalogenitsalz ist.

5. Verfahren nach Anspruch 3, in dem das organische Lösungsmittel ausgewählt ist aus der Gruppe bestehend aus Methanol, Äthanol, 1-Propanol, 1-Butanol, 2-Propanol, 2-Butanol, Acetonitril und Tetrahydrofuran.

6. Verfahren nach Anspruch 3, in dem das Volumsverhältnis von Lösungsmittel zu Wasser im Bereich von 5 : 95 bis 95 : 5 liegt.

7. Verfahren nach Anspruch 3, in dem das Gewichtsverhältnis von Anilin zu Wasser und Lösungsmittel enthaltender Mischung im Bereich von 0,001 bis 1,20 liegt.

8. Verfahren nach Anspruch 3, in dem das Molverhältnis von Oxidationsmittel zu primärem Amin im Bereich von 4 : 1 bis 1 : 4 liegt.

**9.** Verfahren nach Anspruch 3, in dem das Molverhältnis von Base zu Oxidationsmittel im Bereich von 1 : 1 bis 4 : 1 liegt.

**10.** Verfahren nach Anspruch 3, in dem der Oxidationsschritt bei einer Temperatur von weniger als 35°C durchgeführt wird.

**11.** Verfahren nach Anspruch 3, in dem das Reduktionsmittel ausgewählt ist aus der Gruppe bestehend aus einem ein Palladium-auf-Kohle-Katalysator und einem Platin-auf-Kohle-Katalysator.

**12.** Verfahren nach Anspruch 1, in dem das primäre aromatische Amin Anilin und das organische Lösungsmittel ein Alkohol ausgewählt aus Methanol, Äthanol, 1-Propanol und 2-Propanol ist und die katalytische Reduktion zum Herstellen von 4-Aminodiphenylamin oder 2-Aminidiphenylamin oder einer Mischung hievon verwendet wird.

## Revendications

**1.** Procédé pour la préparation d'amines aromatiques substituées répondant à la formule :

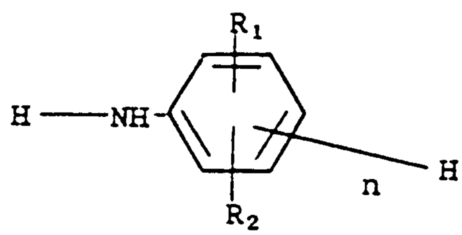

dans laquelle n a une valeur de 2 à 5,
$R_1$ et $R_2$ représentent des radicaux aliphatiques identiques ou différents ou l'hydrogène, qui comprend (1) la mise en contact d'une ou plusieurs amines aromatiques primaires avec un agent oxydant hypohalogénite dans un mélange contenant de l'eau, un solvant organique et une base, et (2) la réduction de la solution à l'aide d'un agent réducteur pour produire l'amine aromatique substituée.

**2.** Procédé selon la revendication 1, dans lequel les amines aromatiques primaires présentent la structure :

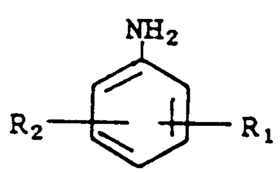

dans laquelle $R_1$ et $R_2$ représentent des radicaux aliphatiques identiques ou différents ou l'hydrogène.

**3.** Procédé selon la revendication 2, dans lequel l'amine aromatique primaire est l'aniline.

**4.** Procédé selon la revendication 1, dans lequel l'agent oxydant est un sel hypohalogénite.

**5.** Procédé selon la revendication 3, dans lequel le solvant organique est choisi dans le groupe constitué par le méthanol, l'éthanol, le 1-propanol, le 1-butanol, le 2-propanol, le 2-butanol, l'acétonitrile et le tétrahydrofuranne.

**6.** Procédé selon la revendication 3, dans lequel le rapport volumique du solvant à l'eau est compris entre 5:95 et 95:5.

7. Procédé selon la revendication 3, dans lequel le rapport pondéral de l'aniline au mélange contenant l'eau et le solvant est compris entre 0,001 et 1,20.

8. Procédé selon la revendication 3, dans lequel le rapport molaire de l'agent oxydant à l'amine primaire est compris entre 4:1 et 1:4.

9. Procédé selon la revendication 3, dans lequel le rapport molaire de la base à l'agent oxydant est compris entre 1:1 et 4:1.

10. Procédé selon la revendication 3, dans lequel on met en oeuvre l'étape d'oxydation à une température de moins de 35 ° C.

11. Procédé selon la revendication 3, dans lequel l'agent de réduction est choisi dans le groupe constitué par un catalyseur de palladium sur carbone et un catalyseur de platine sur carbone.

12. Procédé selon la revendication 1, dans lequel l'amine aromatique primaire est l'aniline, le solvant organique est un alcool choisi parmi le méthanol, l'éthanol, le 1-propanol et le 2-propanol et dans lequel on utilise une réduction catalytique pour produire la 4-aminodiphénylamine ou la 2-aminodiphénylamine ou leur mélange.